# EUROPEAN PATENT APPLICATION

(11) **EP 4 803 623 A1**
(43) Date of publication of application: **09.09.2026**
(21) Application number: 26160906.9
(22) Date of filing: 26.02.2026
(51) Int. Cl.: C12N 9/22, C07K 14/195

(54) **USE OF HIGH-ACTIVITY AND THERMOSTABLE RIBONUCLEASE SIRE_0902 IN SPECIFIC RECOGNITION OF GG SITES**

(30) Priority: 05.03.2025 CN 202510256486
(71) Applicant: BestEnzymes Biotech Co., Ltd., Lianyungang, Jiangsu 222005 (CN)
(72) Inventor: JIANYUN, Yao, Guangzhou City, Guangdong Province 510301 (CN); HENGHAO, Xu, Lianyungang City, Jiangsu Province 222005 (CN); XIAOXUE, Wang, Guangzhou City, Guangdong Province 510301 (CN); CHEN, Lu, Lianyungang City, Jiangsu Province 222005 (CN); XU, Feng, Qingdao City, Shandong Province 266237 (CN); QUNXIN, She, Qingdao City, Shandong Province 266237 (CN); HONGJIE, Ren, Lianyungang City, Jiangsu Province 222005 (CN); XUNING, Zhang, Lianyungang City, Jiangsu Province 222005 (CN); RUI, Deng, Lianyungang City, Jiangsu Province 222005 (CN); ZUNFANG, Gao, Lianyungang City, Jiangsu Province 222005 (CN)
(74) Representative: Murgitroyd & Company

(57) **Abstract**

Disclosed is use of high-activity and thermostable ribonuclease SiRe_0902 in specific recognition of GG sites, where an amino acid sequence thereof is shown in SEQ ID NO. 2, and a nucleotide sequence thereof is shown in SEQ ID NO. 1. The ribonuclease SiRe_0902 has very efficient and specific RNA hydrolytic activity and does not exhibit any nonspecific cleavage of DNA. In addition, the enzyme is derived from a thermophilic archaeon and has been demonstrated to possess good thermostability, exhibiting very high RNA cleavage activity at both 37°C and 65°C. It was demonstrated that the enzyme can specifically recognize and cleave GG sequences, and also exhibits high cleavage activity toward GG sequences located in regions with secondary structures. Therefore, ribonuclease SiRe_0902 is a novel thermostable ribonuclease with extremely high RNA hydrolytic activity under both normal and high-temperature conditions, meeting the needs of clinical, industrial production, and molecular cloning fields.

## Description

### CROSS-REFERENCES TO RELATED APPLICATIONS

This application claims the priority benefit of China application serial no. 202510256486.3, filed on March 5, 2025. The entirety of the above-mentioned patent application is hereby incorporated by reference herein and made a part of this specification.

### BACKGROUND

### Technical Field

The present disclosure belongs to the fields of biochemical engineering and biotechnology, and in particular to use of a high-activity and thermostable ribonuclease SiRe_0902 in specific recognition of GG sites under high-temperature conditions.

### Description of Related Art

Ribonucleases (RNases) refer to enzymes capable of hydrolyzing phosphodiester bonds in RNA, and different RNases have different specificities. Currently, commonly used ribonucleases include RNase A, RNase H, RNase T1, and the like. Ribonuclease is a globular protein that is soluble in water and 50% acetone. The enzyme has an optimal temperature of 60°C, and loses its activity above 85°C, with an optimal pH of 7.6. Specifically, ribonuclease A (RNase A) is derived from bovine pancreas and is an endoribonuclease that specifically attacks the 3' terminus of pyrimidine residues in RNA, cleaving phosphodiester bonds formed between cytosine/uracil and adjacent nucleotides. Final reaction products are 3'-pyrimidine nucleotides and oligonucleotides with a 3'-pyrimidine nucleotide at the terminus. In the absence of cofactors and divalent cations, the activity of ribonuclease A can be inhibited by placental ribonuclease inhibitor (RNasin) or ribonucleoside vanadyl complex (VRC). Ribonuclease T1 (RNase T1) is derived from Aspergillus oryzae and specifically acts on the 3' phosphate of guanine residues, cleaving the phosphodiester bond between the 3' phosphate of guanine and the 5' hydroxyl group of an adjacent nucleotide. Final reaction products are 3'-guanosine monophosphate and oligonucleotide fragments with a 3'-guanosine monophosphate at the terminus. Ribonuclease H (RNase H) was first discovered in calf thymus tissue, and its coding gene has been cloned into *Escherichia coli.* It specifically degrades the RNA strand in DNA:RNA hybrid duplexes, producing oligonucleotides and mononucleotides having 3'-OH and 5'-phosphate termini, and it cannot degrade single-stranded or double-stranded DNA or RNA.

At present, ribonucleases have been widely used in various fields, including molecular cloning, clinical practice, food and pharmaceuticals. In molecular cloning, ribonucleases can be used to: (1) remove RNA molecules from DNA:RNA hybrids or DNA preparations; (2) determine the positions of single-base mutations in RNA or DNA; and (3) detect RNA in the RNase protection assay. In addition, it has been reported that ribonucleases can alter host cell metabolism, inhibit virus synthesis, suppress influenza virus proliferation in vitro, and inhibit the formation of vaccinia virus and herpesvirus in chick embryos. Clinically, intramuscular injection of 180 mg of ribonuclease per day is beneficial for the treatment of epidemic encephalitis. However, the types of ribonucleases currently available on the market are limited, and in particular, commercialization of thermostable RNA endonucleases with sequence recognition sites is almost nonexistent, resulting in a great demand for ribonucleases with novel functions.

### SUMMARY

The present disclosure aims to provide use of a highly efficient and thermostable ribonuclease SiRe_0902 in specific recognition of GG sites. The ribonuclease SiRe_0902 is directly prepared from the thermophilic archaeon *Sulfolobus islandicus* REY15A and is a highly efficient, heat-resistant, and stable ribonuclease capable of specifically recognizing and cleaving GG sites in RNA sequences, and can efficiently cleave RNA with secondary structures at 37°C or 65°C. This provides very promising application prospects for the analysis of RNA with complex structures. Importantly, this enzyme has a stable structure and remains highly active after being stored at 4°C for one year, thus possessing the characteristics for development into a commercial RNA endonuclease.

A first objective of the present disclosure is to provide use of a ribonuclease SiRe_0902 in specific recognition and cleavage of GG sites in RNA, where an amino acid sequence of the ribonuclease SiRe_0902 is shown in SEQ ID NO.2.

Preferably, a nucleotide sequence of gene encoding the ribonuclease SiRe_0902 is shown in SEQ ID NO.1.

The ribonuclease gene SiRe_0902 of the present disclosure is derived from the thermophilic archaeon *Sulfolobus islandicus* REY15A. Through molecular cloning and activity verification methods, the present disclosure identified that the gene SiRe_0902 encodes a highly efficient and thermostable ribonuclease. Under normal conditions, this enzyme is modified and silenced by a nucleic acid-specific enzyme encoded by the downstream gene SiRe_0903. Experimental verification showed that when the ribonuclease gene SiRe_0902 is co-expressed and purified with the downstream gene SiRe_0903, there is no effect on host growth, and there is no stable interaction between them. Therefore, a large amount of nucleic acid-modified ribonuclease SiRe_0902 may be purified.

Preferably, the cleavage of GG sites in RNA is cleavage of a phosphodiester bond in a middle of a GG sequence.

Preferably, a concentration of the ribonuclease SiRe_0902 is 5-10 µg/mL.

Preferably, a concentration of the ribonuclease SiRe_0902 is 5 µg/mL.

Preferably, a reaction temperature for the specific recognition and cleavage of GG sites in RNA is 37-70°C, and a reaction duration is 10-20 min.

Preferably, the reaction temperature for the specific recognition and cleavage of GG sites in RNA is 65°C, and the reaction duration is 12-15 min; or the reaction temperature for the specific recognition and cleavage of GG sites in RNA is 37°C, and the reaction duration is 20-30 min. More preferably, the reaction temperature for the specific recognition and cleavage of GG sites in RNA is 65°C, and the reaction duration is 12 min.

A second objective of the present disclosure is to provide use of a ribonuclease SiRe_0902 in hydrolysis of RNA in non-disease diagnosis and treatment, where an amino acid sequence of the ribonuclease SiRe_0902 is shown in SEQ ID NO.2.

Preferably, a concentration of the ribonuclease SiRe_0902 is 5-10 µg/mL, a hydrolysis temperature is 37-65°C, and a hydrolysis duration is 10-20 min.

Preferably, the concentration of the ribonuclease SiRe_0902 is 10 µg/mL, the hydrolysis temperature is 65°C, and the hydrolysis duration is 15 min.

### The present disclosure has the following advantages:

The ribonuclease SiRe_0902 provided by the present disclosure has very efficient and specific RNA hydrolytic activity and does not exhibit any nonspecific cleavage of DNA. In addition, the enzyme is derived from a thermophilic archaeon and has been demonstrated to possess good thermostability, exhibiting very high RNA cleavage activity at both 37°C and 65°C. Therefore, ribonuclease SiRe_0902 is a novel thermostable ribonuclease with extremely high RNA hydrolytic activity and substrate sequence dependence under both normal and high-temperature conditions, meeting the needs of clinical, industrial production, and molecular cloning fields.

Ribonuclease SiRe_0902 can specifically recognize and cleave GG sites in RNA sequences. At a temperature of 65°C, when a concentration of the RNA endonuclease SiRe_0902 is 5 µg/mL and a reaction duration is within 15 min, cleavage of the substrates is completely sequence-dependent, and no random sequence hydrolysis activity is observed. The SiRe_0902 has the potential for development into a ribonuclease product, and can recognize and cleave GG sequences under high-temperature conditions, exhibiting significant advantages in opening RNA structures.

The thermophilic archaeon *Sulfolobus islandicus* REY15A is a model organism and has been disclosed in the literature: Genome analyses of Icelandic strains of *Sulfolobus islandicus,* model organisms for genetic and virus-host interaction studies. The applicant also possesses this strain and guarantees its public availability within 20 years from the filing date.

### BRIEF DESCRIPTION OF THE DRAWINGS

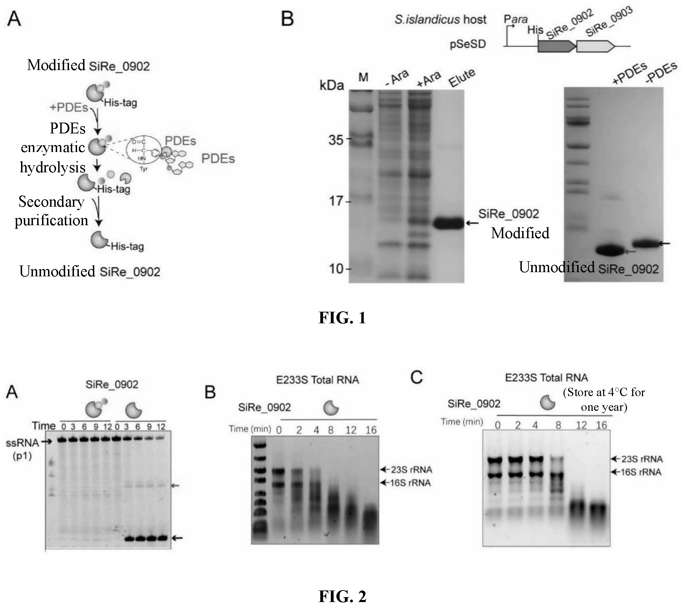

FIG. 1: A illustrates a process for preparing unmodified active ribonuclease SiRe_0902. B illustrates an SDS-PAGE electrophoresis gel image of a ribonuclease SiRe_0902, where in the left electrophoresis gel image, lane 1 (lane 1 refers to a first band to the right of the Marker) illustrates crude protein of REY15A containing pSeSD-SiRe_0902-0903-NHis without L-Ara induction, lane 2 illustrates total cellular protein after L-Ara induction, and lane 3 illustrates purified SiRe_0902 protein (nucleic acid-modified); and the right electrophoresis gel image illustrates molecular weight changes of a ribonuclease SiRe_0902 before and after PDEs treatment.
FIG. 2 illustrates the effect of reaction time on the activity of a ribonuclease SiRe_0902 (10 µg/mL) in cleaving an RNA probe (A), and total RNA samples (B), as well as activity detection of a ribonuclease SiRe_0902 after storage at 4°C for one year (C).

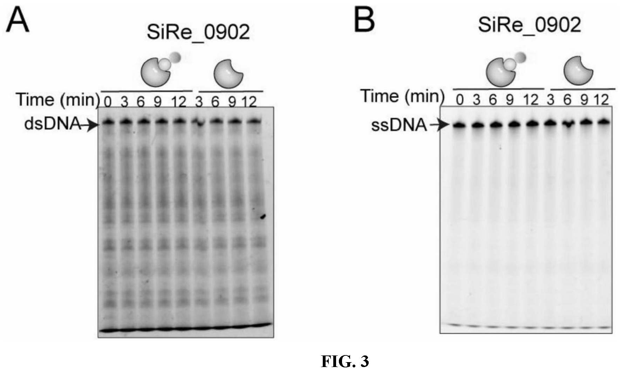

FIG. 3 illustrates the detection of ability of a ribonuclease SiRe_0902 (10 µg/mL) to cleave a single-stranded DNA probe (A) and a double-stranded DNA probe (B).

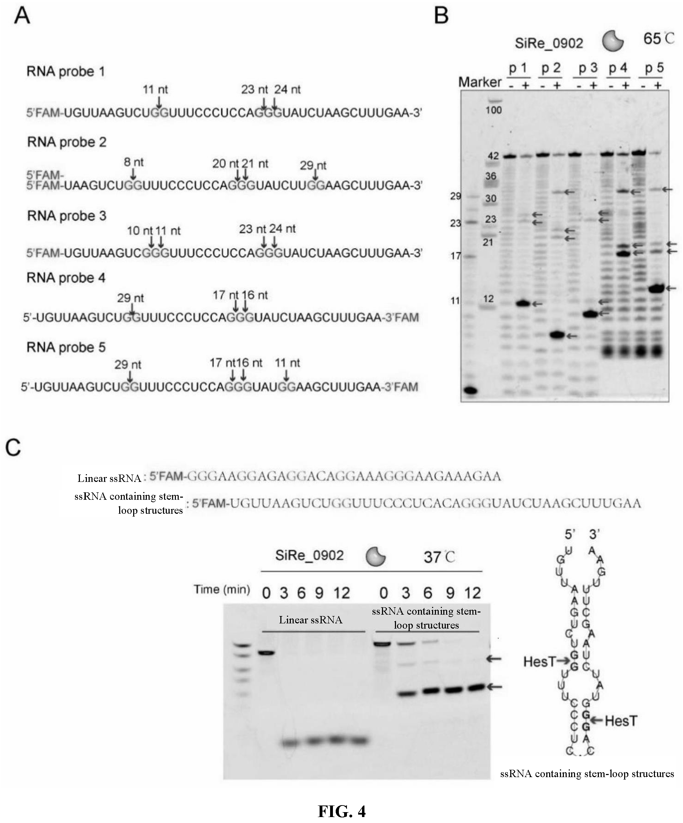

FIG. 4 illustrates identification results of sequences recognized and cleaved by ribonuclease SiRe_0902 (5 µg/mL), where (A) illustrates a reaction temperature of 65°C and a reaction duration of 10 min; (B) illustrates a reaction temperature of 37°C and a reaction duration of 10 min; and (C) illustrates complete hydrolysis of GG sequences in RNA at room temperature.

### DESCRIPTION OF THE EMBODIMENTS

The following embodiments further illustrate the present disclosure and are not intended to limit the present disclosure.

### Example 1: Cloning and vector construction of ribonuclease SiRe_0902

A pSeSD plasmid (disclosed in the literature "A synthetic arabinose-inducible promoter confers high levels of recombinant protein expression in thermophilic archaeon *Sulfolobus islandicus")* is a high-copy-number expression vector for the thermophilic archaeon *Sulfolobus islandicus* REY15A and is commonly used for intracellular expression and purification of target proteins. Genomic DNA of wild-type *Sulfolobus islandicus* REY15A was extracted using a Tiangen kit and used as a template for subsequent PCR cloning. Primers were designed to amplify an SiRe_0902-0903 operon (a nucleotide sequence of SiRe_0902 is shown in SEQ ID NO. 1; and a nucleotide sequence of SiRe_0903 is shown in SEQ ID NO. 3). The primer sequences were as follows: forward primer SiRe_0902-his-F(pSeSD): ( SEQ ID NO. 5 ) 5'GGAATTCCATATGCACCACCACCACCACCACAACTATAAAGAATG GATAGAG 3'; and reverse primer: SiRe_0903-R(pSeSD) : ( SEQ ID NO. 6 ) 5'ACGCGTCGACTTATAGAAATTCCTTTATATCT 3'. SiRe_0902-his-F(pSeSD) and SiRe_0903-R(pSeSD) were used as primers, the genomic DNA of wild-type *Sulfolobus islandicus* REY15A was used as a template, a coding region of the SiRe_0902-0903 operon were amplified by PCR using PrimeSTAR DNA polymerase (Takara Bio). A PCR reaction system is shown in Table 1. PCR conditions were as follows: 95°C for 5 min; followed by 30 cycles of 95°C for 30 s, 56°C for 30 s, and 72°C for 30 s; and finally 72°C for 10 min. After the reaction was completed, the resulting PCR amplification product was subjected to 1.0% agarose gel electrophoresis, and a target gene fragment of approximately 700 bp was recovered and purified. The recovered fragment was subjected to double digestion with restriction enzymes NdeI and Sall, and the digested products were subjected to 1.0% agarose gel electrophoresis, followed by gel extraction and purification. The fragment was then ligated with the vector pSeSD, which had also been digested with NdeI and Sall using T4 DNA ligase (BestEnzymes Biotech, China). The ligation product was transformed into competent cells of *Escherichia coli* BW25113, and positive transformants were screened. PCR verification was performed, and sequencing was carried out by a sequencing company, thereby obtaining a recombinant plasmid pSeSD-SiRe_0902-0903-NHis and a target strain containing the recombinant plasmid.

**Table 1 PCR Reaction System**

| Component | Volume |
|---|---|
| *Pfu* enzyme (5 U/µL) | 0.5 µL |
| 5×buffer(Mg²⁺ plus) | 2.5 µL |
| dNTP Mixture (each 2.5 mM) | 2.5 µL |
| DNA template | 10 ng |
| Forward primer (10 µM) | 1 µL |
| Reverse primer (10 µM) | 1 µL |
| Sterile ddH₂O | up to 25 µL |

### Example 2: High-efficiency expression of ribonuclease SiRe_0902

### 2.1 Preparation of competent cells of Sulfolobus islandicus REY15A

1. On the day before preparing competent cells, 2 mL of *Sulfolobus islandicus* REY15A starter culture was inoculated into 100 mL of SCVU (Uracil, 10 µg/mL) medium, and cultured overnight at 78°C.
   SCVU: Uracil (2 mg/mL stock) was added at 10 µL/mL to 1 mL of SCV (Table 3).
2. Cultivation was stopped when OD₆₀₀ reached 0.2 (for ease of operation, an OD₆₀₀ between 0.2 and 0.3 is also acceptable), and the culture was transferred at room temperature into 2 × 50 mL centrifuge tubes, which were then centrifuged at 6,000 rpm at room temperature for 10 min to collect cells. A supernatant (all supernatant removed by aspiration) was discarded.
3. 15 mL of room-temperature sucrose solution (20 mM) was added to each tube, gently pipetted up and down to resuspend the cells. Centrifugation was performed at 6,000 rpm at room temperature for 10 min, and repeated twice.
4. The cells were resuspended in room-temperature sucrose solution (20 mM) (not vortex) to adjust OD₆₀₀ of the competent cells to 5-10. The competent cells were stored at room temperature for one week or stored at -80°C.

### 2.2 Electroporation of the target recombinant plasmid into the expression strain REY15A

1. 0.8 µg (0.5-1 µg) of the recombinant plasmid pSeSD-SiRe_0902-0903-NHis obtained in Example 1 was taken and mixed with 50 µL of competent cells of the *Sulfolobus islandicus* REY15A, and incubated at room temperature for 30 min.
2. 50 µL of the competent cell-DNA suspension was transferred into a corresponding electroporation cuvette. Electroporation conditions were as follows: 1.2 kV, 600 Ω, 25 µF (Bio-Rad Gene Pulser II), with a time constant of 12 ms.
3. The transformed cells were transferred into a 1.5 mL centrifuge tube containing 800 µL of incubation medium (see Table 2) and preheated to 75°C. After incubation at 75°C for 1 h (without shaking), the sample was removed and placed at room temperature until plating.
4. The required 0.4% Gelrite (Sigma, CAS No.: 71010-52-1) solution and 2× SCV, were heated and mixed at a volume ratio of 1:1 to obtain 1× SCV top agar. 6 mL of the 1× SCV top agar was added to 50 µL of the electroporated cells and mixed well to obtain a mixture, and the mixture was added onto plates preheated and coated with bottom agar SCV, allowed to solidify at room temperature for 30 min, and then cultured at 75°C for 7 days to check transformants.

Bottom agar: It was prepared as shown in Tables 2-4, by mixing 1.4% Gelrite solution with an equal volume of 2× SCV.

Top agar: It was prepared by mixing 0.4% Gelrite with 2× SCV at equal volumes, with final concentrations of 0.2% and 1× SCV, respectively.

### 2.3 Protein induction and expression

The *Sulfolobus islandicus* REY15A expression strain containing pSeSD-SiRe_0902-0903-Nhis was cultured in SCV medium until OD₆₀₀ reached approximately 0.5, an inducer D-arabinose was added to a final concentration of 10 mM, and then cultured at 75°C for 48 h. 500 mL of bacterial culture was centrifuged at 4,500 rpm at room temperature for 10 min to collect bacterial cells, the bacterial cells were resuspended in 30 mL of PBS buffer (50 mM, pH 7.4), subjected to ultrasonic disruption at 250 W with 5 s on/5 s off pulses for a total of 10 min, and then centrifuged at 10,000 rpm at 4°C for 30 min to collect a supernatant.

### 2.4 Protein purification and SDS-PAGE electrophoresis

The supernatant collected in the step 2.3 was purified using a nickel ion affinity chromatography column, with specific procedure as follows: the column was first eluted with 5 column volumes of 10 mM imidazole, followed by 20-30 column volumes of 20 mM imidazole, and then 1 column volume of 50 mM imidazole, and finally eluted with 2.5 mL of 300 mM imidazole, and 2.5mL of final eluate was collected. Desalting was performed using a Sephadex G-25 desalting column. Specific operating instructions were subject to the instruction manual of GE Company (Cytiva). The purified expression product was subjected to SDS-PAGE gel electrophoresis to obtain purified nucleic acid-modified ribonuclease SiRe_0902 (A of FIG. 1). The purified protein had a size of approximately 15 kDa, which is consistent with theoretical expectations.

**Table 2 Mineral Salt Solution (pH 3.5)**

| Component | Content | |
|---|---|---|
| Ammonium Sulfate | 3 g/L | Incubating the medium, natural pH (approximately 5.5). |
| Kalium Sulfate | 0.5 g/L | |
| Kalium Chlorid | 0.1 g/L | |
| Glycine | 0.7 g/L | |
| 1% MnCl₂·4H₂O | 80 µL/L | |
| 1% Na₂B₄O₇·10H₂O | 210 µL/L | |
| 1% ZnSO₄·7H₂O | 11 µL/L | |
| 0.1% CuSO₄·5H₂O | 25 µL/L | |
| 0.1% Na₂MoO₄·2H₂O | 15 µL/L | |
| 0.1% VOSO₄·5H₂O | 15 µL/L | |
| 0.1% CoSO₄·7H₂O | 5 µL/L | |
| 0.1% NiSO₄·6H₂O | 5 µL/L | |
| 1% FeSO₄·7H₂O; 0.5M HCl | 200 µL/L (a total of 566 µL concentrated solution) | |
| 1M MgCl₂ · 6H₂O | 1 mL/L | Add 20 mL/L to 2× medium |
| 0.3M Ca(NO₃)₂·4H₂O | - | |

### (Water and concentrated sulfuric acid were added at a ratio of 1:1 for dilution, and pH was adjusted to 3.)

**Table 3 SCV Medium**

| Component | Added amount |
|---|---|
| Mineral Salt Solution (pH 3.5) (Table 2) | 1 L |
| Sucrose (50% stock) | 4 mL/L |
| Casamino Acid (20% stock) (sterilized by filtration); treated with 0.1% activated charcoal | 10 mL/L |
| Vitamin solution (100 × stock) (Table 4) | 10 mL/L |
| 10% YEAST EXTRACT | 250 µL/L |

**Table 4 Vitamin Mixture 100×**

| Component | Content/L |
|---|---|
| Niacin | 10 mg |
| Biotin | 4 mg |
| Pantothenate | 10 mg |
| Lipoic acid | 10 mg |
| Folic acid | 4 mg |
| p-Aminobenzoic acid | 10 mg |
| Vitamin B1 (thiamin, thiamine) | 10 mg |
| Vitamin B2 (Riboflavin) | 10 mg |
| Vitamin B6 (Pyridoxine) | 10 mg |
| Vitamin B12 (cobalamin) | 10 mg |

### (Aqueous stock solution, and sterilized by filtration.)

### Example 3: Preparation of active ribonuclease SiRe_0902

The ribonuclease SiRe_0902 (SiRe_0902-modified) purified in the step 2.4 of Example 2 was processed according to the preparation procedure for active ribonuclease SiRe_0902 (A of FIG. 1). First, 2.5 mL of desalted protein SiRe_0902-modified was mixed with 100 µL of phosphodiesterase (PDE, 2 U µL⁻¹) and incubated overnight (12-16 h) at 4°C. After the treatment was completed, the protein mixture was re-purified using a nickel ion affinity chromatography column, and the purification steps were the same as those in the step 2.4 of Example 2. After purification, small nucleic acid molecules and phosphodiesterase (PDE) were filtered out, and finally nucleic acid-unmodified ribonuclease SiRe_0902 was collected. The purified product was subjected to SDS-PAGE gel electrophoresis, and the purified protein had a size of approximately 15 kDa (B of FIG. 1), which is consistent with theoretical expectations.

### Example 4: Activity assay of ribonuclease SiRe_0902

To ensure the accuracy of the experimental results, activities of a ribonuclease SiRe_0902 in two states were compared in this study. First, protein concentrations of the two states of SiRe_0902 (nucleic acid-modified and nucleic acid-unmodified) were determined using a BCA protein quantification kit (P0010, Beyotime Technology). Stock solution concentrations of both were adjusted to 0.3 mg/mL using a Tris-HCl (20 mM, pH 8.0) buffer for later use. In this study, four commonly used nucleic acid substrates were selected for testing, including a single-stranded RNA probe, total RNA, a single-stranded DNA probe, and a double-stranded DNA probe. Probe sequences are shown in Table 5.

**Table 5 Probe Sequence Listing**

| Probe | Sequence |
|---|---|
| RNA probe 1 ( SEQ ID NO. 7 ) | 5’FAM- UGUUAAGUCUGGUUUCCCUCCAGGGAUCUAAGCUUGAACCCCC CCC 3’ |
| sDNA probe ( SEQ ID NO. 8 ) | 5'FAM- TTCGAGCTCGGTACCCGGTTGATAGGGAACAAGACGAATAGGGA TCCTCTAGAGTCGAC |
| dsDNA probe ( SEQ ID NO. 9 ) | 5'FAM- TTCGAGCTCGGTACCCGGTTGATAGGGAACAAGACGAATAGGGA TCCTCTAGAGTCGAC AAGCTCGAGCCATGGGC CAACTATCCTGTTCTGCTTATCCCTAGGAGATCTCGAGCTG |

Reaction conditions for hydrolysis of substrates by ribonuclease SiRe_0902 were as follows: (1) The two SiRe_0902 proteins were serially diluted to concentrations of 50, 100, and 500 µg/mL, respectively, and the dilution buffer was 50 mM Tris-HCl, pH 7.5, 10 mM MgCl₂. (2) 1 µL of a ribonuclease SiRe_0902 was added to a 10 µL reaction system, with a concentration of nucleic acid substrate at 50 nM, and the resulting mixture system was reacted at 60°C for 15 min. (3) Detection of substrate hydrolysis: 2 µL of nucleic acid loading buffer was added to the 10 µL reaction system, followed by electrophoresis on a 15% urea gel at 110 V for 30 min, and FAM signal exposure detection was performed (FIGS. 2-3).

The results in FIGS. 2 and 3 show that SiRe_0902 nucleic acid-modified has no ribonuclease activity and has no effect on RNA or DNA probes. In contrast, nucleic acid-unmodified SiRe_0902 has high-efficiency ribonuclease activity and can specifically cleave RNA substrates at 65°C with high cleavage efficiency. Even at extremely low enzyme concentrations (10 µg/mL), substrate hydrolysis can be completed within 15 min. In addition, the activity of freshly prepared and unmodified SiRe_0902 was compared with that of SiRe_0902 stored at 4°C for one year, and the results showed that it still retained high RNA cleavage activity, indicating good stability (C of FIG. 2).

### Example 5: Specificity detection of recognition sequences of ribonuclease SiRe_0902

According to the reaction conditions of Example 4 (with the reaction temperature changed to 65°C), SiRe_0902 exhibits certain sequence selectivity when cleaving RNA substrates. 50 mM Tris-HCl, pH 7.5, and 10 mM MgCl₂ were used as a buffer, five probes with different sequences labeled with FAM at the 5' terminus or 3' terminus (A of FIG. 4) were introduced to detect the recognition sequence of SiRe_0902. The in vitro activity assay results showed that SiRe_0902 specifically recognizes and cleaves internal GG sequences in RNA probes. At 65°C, when a concentration of the RNA endonuclease SiRe_0902 was 5 µg/mL and a reaction duration was within 15 min, cleavage of the substrates was completely sequence-dependent, and no random sequence hydrolysis activity was observed (B of FIG. 4). The above results indicate that the thermostable endonuclease SiRe_0902 has the potential for development into a ribonuclease product, and can recognize and cleave GG sequences under high-temperature conditions, exhibiting significant advantages in opening RNA structures.

Given that the conventional enzyme reaction temperature is 37°C, the activity of SiRe_0902 at 37°C (with other reaction conditions the same as those in Example 4) was further tested. The results showed that SiRe_0902 at room temperature can not only hydrolyze single-stranded RNA lacking secondary structure, but also cleave RNA molecules with complex secondary structures, thereby achieving complete hydrolysis of GG sequences in RNA at room temperature (C of FIG. 4). SEQ ID NO. 1 (Nucleotide sequence of ribonuclease gene SiRe_0902).

ATGAACTATAAAGAATGGATAGAGCAAGCTTTGGAAGATCTCGACACTGCAA AACTTCTTTTAACTAATGGGAAATATTACGCTTCTGCCTTCTATTCTCAGCAAGCAGTA GAGAAATCATTAAAGTCCCTTATAATTTATCTTGGAAAAGATCCTGGTAAGACGCATTC ACTTACTGAACTTATTGAAATGGTAGAGAAGGAAGGAGTGACTATGCCAATAAATATT AAAGAGAATCTGATGGTTCTTTCACCCCATTTCATAATTTCAAGATATCCAGACGCTG CAAACGGAGTTCCCTTTAAGCAGTATAGTAAATCAATTTCTGAGGACCTTTATAATAG AGCAAAAGAGGTGATTGAATGGGTAAAGGAAAATCTGCAATAGSEQ ID NO. 2 (Amino acid sequence of ribonuclease SiRe_0902).

MNYKEWIEQALEDLDTAKLLLTNGKYYASAFYSQQAVEKSLKSLIIYLGKDPG KTHSLTELIEMVEKEGVTMPINIKENLMVLSPHFIISRYPDAANGVPFKQYSKSISEDLYN RAKEVIEWVKENLQSEQ ID NO. 3 (Nucleotide sequence of ribonuclease SiRe_0903).

ATGGGTAAAGGAAAATCTGCAATAGAGAGCCAAATGAAGTTAATAAATCTAG TAAAGGAAATAGTGGAAGAAATAGCTAAGGACTTCCAGCAGTTAGACGAAGTTTATA TCTTTGGCTCTAGAGCTAAAGGAAATTATTTAGATACTAGTGATATAGATGTTATCTTC GTTTTCAAGGGCATAAAGGAAATGAACGTATTTGATAGGATGTATATGGTAAGTAAAT ACATAAAAGGAAATATAGATTATATAGTATTAAACGAGGACGAAAAGGATAGAATAAG AGAGAAGAAATTATTTTGGAAGAGGAATAAGGGATTTGTAGATATAAAGGAATTTCTA TAASEQ ID NO. 4 (Amino acid sequence of ribonuclease SiRe_0903).

MGKGKSAIESQMKLINLVKEIVEEIAKDFQQLDEVYIFGSRAKGNYLDTSDIDV
IFVFKGIKEMNVFDRMYMVSKYIKGNIDYIVLNEDEKDRIREKKLFWKRNKGFVDIKEF
L.

## Claims

1. Use of a ribonuclease SiRe_0902 in specific recognition and cleavage of GG sites in RNA in non-disease diagnosis and treatment, wherein an amino acid sequence of the ribonuclease SiRe_0902 is shown in SEQ ID NO.2.

2. The use according to claim 1, wherein a nucleotide sequence of gene encoding the ribonuclease SiRe_0902 is shown in SEQ ID NO. 1.

3. The use according to claim 1, wherein the cleavage of GG sites in RNA is cleavage of a phosphodiester bond in a middle of a GG sequence.

4. The use according to claim 1, wherein a concentration of the ribonuclease SiRe_0902 is 5-10 µg/mL.

5. The use according to claim 4, wherein the concentration of the ribonuclease SiRe_0902 is 5 µg/mL.

6. The use according to claim 1, wherein a reaction temperature for the specific recognition and cleavage of GG sites in RNA is 37-70°C, and a reaction duration is 10-30 min.

7. The use according to claim 6, wherein the reaction temperature for the specific recognition and cleavage of GG sites in RNA is 65°C, and the reaction duration is 12-15 min; or the reaction temperature for the specific recognition and cleavage of GG sites in RNA is 37°C, and the reaction duration is 20-30 min.

8. Use of a ribonuclease SiRe_0902 in hydrolysis of RNA in non-disease diagnosis and treatment, wherein an amino acid sequence of the ribonuclease SiRe_0902 is shown in SEQ ID NO.2.

9. The use according to claim 8, wherein a concentration of the ribonuclease SiRe_0902 is 5-10 µg/mL, a hydrolysis temperature is 37-65°C, and a hydrolysis duration is 10-20 min.

10. The use according to claim 9, wherein the concentration of the ribonuclease SiRe_0902 is 10 µg/mL, the hydrolysis temperature is 65°C, and the hydrolysis duration is 15 min.
